# EUROPÄISCHE PATENTSCHRIFT

(11) **EP 1 194 408 B1**
(45) Veröffentlichungstag und Bekanntmachung des Hinweises auf die Patenterteilung: **02.05.2003**
(21) Anmeldenummer: 00942128.0
(22) Anmeldetag: 23.06.2000
(51) Int. Cl.: C07D 231/24, A01N 43/56

(54) **$g(a)-$g(a)' -SUBSTITUIERTE N-ALKYL-3-ALKENYLBENZOYL-PYRAZOL-DERIVATE**
$g(A)-$g(a)'-SUBSTITUTED N-ALKYL-3-ALKENYLBENZOYL-PYRAZOL-DERIVATIVES
DERIVES DE N-ALKYL-3-ALCENYLBENZOYLPYRAZOLES A SUBSTITUTION $G(A)-$g(a)'

(30) Priorität: 09.07.1999 DE 19931881
(43) Veröffentlichungstag der Anmeldung: 10.04.2002
(73) Patentinhaber: BASF AKTIENGESELLSCHAFT, 67056 Ludwigshafen (DE)
(72) Erfinder: NEIDLEIN, Ulf, D-68165 Mannheim (DE); GÖTZ, Norbert, D-67547 Worms (DE); BAUMANN, Ernest, D-67373 Dudenhofen (DE); VON DEYN, Wolfgang, D-67435 Neustadt (DE); KUDIS, Steffen, D-68167 Mannheim (DE); GÖTZ, Roland, D-68809 Neulussheim (DE); LANGEMANN, Klaus, D-67551 Worms (DE); MAYER, Guido, D-67433 Neustadt (DE); MISSLITZ, Ulf, D-67433 Neustadt (DE); WITSCHEL, Matthias, D-67098 Bad Dürkheinen (DE); OTTEN, Martina, D-67069 Ludwigshafen (DE); WESTPHALEN, Karl-Otto, D-67346 Speyer (DE); WALTER, Helmut, D-67283 Obrigheim (DE)
(86) Internationale Anmeldenummer: EP0005857
(87) Internationale Veröffentlichungsnummer: WO01004095

(56) Entgegenhaltungen:
- WO-A-98/45273
- WO-A-98/52926
- US-A- 4 948 887
- US-A- 5 807 806

## Beschreibung

Die vorliegende Erfindung betrifft α,α'-substituierte N-Alkyl-3-alkenylbenzoyl-Pyrazol-Derivate der Formel I 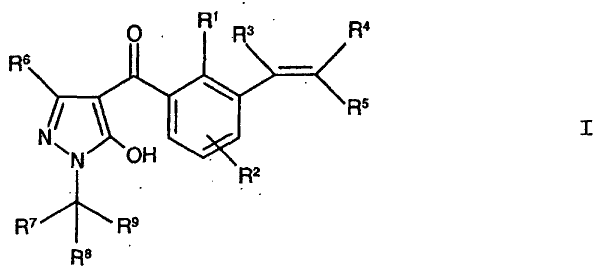 in der die Variablen folgende Bedeutung haben:
- R¹: Halogen, C₁-C₆-Alkyl, C₁-C₆-Alkoxy, C₁-C₆-Halogen-alkyl, C₁-C₆-Alkylthio;
- R²: -S(O)₂R¹⁰, -SO₂OR¹¹,
- R³: Wasserstoff,.
- R⁴, R⁵: Wasserstoff, C₁-C₆-Alkyl,
- R⁶: Wasserstoff;
- R⁷, R⁸, R⁹: Wasserstoff, C₁-C₆-Alkyl, wobei die Alkylgruppe gegebenenfalls ein- oder mehrfach durch Halogen oder Cyano substituiert sein kann, und die Reste R⁷, R⁸ und R⁹ jeweils gleich oder verschieden sind, wobei maximal einer der Reste aus der Gruppe R⁷, R⁸, R⁹ Wasserstoff bedeutet;
- R¹⁰, R¹¹: C₁-C₆-Alkyl,
sowie deren Tautomere oder landwirtschaftlich brauchbaren Salze.

Außerdem betrifft die Erfindung Verfahren zur Herstellung von Verbindungen der Formel I, Mittel welche diese enthalten, sowie die Verwendung der Verbindungen der Formel I und diese enthaltende Mittel im agronomischen Bereich, insbesondere bei der Bekämpfung von unerwünschtem Pflanzenwuchs.

Herbizide Wirkstoffe aus der Klasse der Benzoylpyrazole sind beispielsweise bekannt aus EP-A 282 944; WO 98/42677; WO 98/45273; WO98/50366; WO 98/52926; WO 98/56766; US 5,807,806.

Die herbiziden Eigenschaften dieser Verbindungen sowie die Verträglichkeiten gegenüber Kulturpflanzen können jedoch nur bedingt befriedigen. Es lag daher dieser Erfindung die Aufgabe zugrunde, neue Verbindungen mit verbesserten Eigenschaften zu finden, die als Wirkstoff im Pflanzenschutzbereich, insbesondere als Herbizide, eingesetzt werden können.

Diese Aufgabe wird durch die oben definierten Benzoylpyrazole gelöst, die in 1-Stellung des Pyrazolderviates durch eine verzweigte Alkylgruppe substituiert sind. Diese verzweigte Alkylgruppe ist durch den Substituenten -CR⁷R⁸R⁹ am Pyrazolring charakterisiert.

Ferner wurden sehr gut wirksame herbizide Mittel gefunden, die die Verbindungen I enthalten. Außerdem wurden Verfahren zur Herstellung dieser Mittel und Verfahren zur Bekämpfung von unerwünschtem Pflanzenwuchs mit den Verbindungen I gefunden.

Gegenstand der vorliegenden Erfindung sind auch Stereoisomere der Verbindungen der Formel I. Es werden sowohl reine Stereoisomere als auch Gemische hiervon erfaßt.

Die Verbindungen der Formel I enthalten eine Kohlenstoff-Kohlenstoff-Doppelbindung und liegen daher als E-Isomere oder Z-Isomere oder als E/Z-Isomerengemische vor. Weiterhin können die Verbindungen der Formel I weitere Kohlenstoff- bzw. Kohlenstoff-Stickstoff-Doppelbindungen enthalten. Gegenstand der Erfindung sind sowohl die reinen geometrischen Isomere als auch Gemische hiervon.

Die Verbindungen der Formel I können ebenso je nach Substitutionsmuster ein oder mehrere Chiralitätszentren enthalten und liegen dann als Enantiomeren oder Diastereomerengemische vor. Gegenstand der Erfindung sind sowohl die reinen Enantiomeren oder Diastereomeren als auch deren Gemische.

Die Verbindungen der Formel I können ebenso in Form ihrer Tautomeren bzw. als Tautomerengemische vorliegen. Die tautomeren Formen ergeben sich insbesondere durch den Hydroxysubstituenten am Pyrazolring. Insofern können die Verbindungen sowohl als 5-Hydroxypyrazole oder als 5-Oxo-pyrazolinone bezeichnet werden.

Gegenstand der vorliegenden Erfindung sind auch sogenannte Vorläuferverbindungen (precursors), aus denen Verbindungen I durch chemische Umwandlung oder biologischen Abbau resultieren. Derartige Vorläuferverbindungen von Verbindungen I sind beispielsweise Ester oder Ether-Derivate von funktionellen Hydroxygruppen.

Die Verbindungen der Formel I können auch in Form ihrer landwirtschaftlich brauchbaren Salze vorliegen, wobei es auf die Art des Salzes in der Regel nicht ankommt. Im allgemeinen kommen die Salze derjenigen Kationen oder die Säureadditionssalze derjenigen Säuren in Betracht, deren Kationen, beziehungsweise Anionen, die herbizide Wirkung der Verbindungen I nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl oder Hydroxy-C₁-C₄-alkyl und/oder ein Phenyl oder Benzyl ersetzt sein können, vorzugsweise Diisopropylammonium, Tetramethylammonium, Tetrabutylammonium, Trimethylbenzylammonium, des weiteren Phosphoniumionen, Sulfoniumionen, vorzugsweise Tri(C₁-C₄-alkyl)-sulfonium und Sulfoxoniumionen, vorzugsweise Tri(C₁-C₄-alkyl)-sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

Erfindungswesentlich im Sinne der vorliegenden Erfindung sind solche Verbindungen der Formel I, die in 1-Stellung des Pyrazolrings durch eine verzweigte Alkylgruppe -CR⁷R⁸R⁹ substituiert sind. Dies sind insbesondere solche Verbindungen I, in denen maximal einer der Reste aus der Gruppe R⁷, R⁸, R⁹ Wasserstoff bedeutet, und die beiden übrigen Reste von Wasserstoff verschieden sind (z.B. R⁷ = H und R⁸ ≠ H, R⁹ ≠ H).

Die für die Substituenten R¹-R¹¹ genannten organischen Molekülteile stellen Sammelbegriffe für individuelle Aufzählungen der einzelnen Gruppenmitglieder dar. Sämtliche Kohlenwasserstoffketten, also alle Alkyl-, Halogenalkyl-, Cycloalkyl-, Alkoxyalkyl-, Alkoxy-, Halogenalkoxy-, Cycloalkoxy, Alkylthio, Alkylsulfonyl-, Halogenalkylsulfonyl, Alkylcarbonyl-, Halogenalkylcarbonyl, Alkoxycarbonyl-, Alkenyl-, Cycloalkenyl-, Alkinyl-, Alkenyloxy-, Alkinyloxy-Teile können geradkettig oder verzweigt sein. Sofern nicht anders angegeben trägen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₂-C₄-Alkyl: Ethyl, n-Propyl, 1-Methylethyl, Butyl, 1-Methylpropyl, 2-Methylpropyl oder 1,1-Dimethylethyl;
- C₁-C₄-Alkyl: C₂-C₄-Alkyl, wie voranstehend genannt sowie Methyl;
- C₂-C₆-Alkyl; C₂-C₄-Alkyl, wie voranstehend genannt, sowie Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1-Ethyl-1-methylpropyl oder 1-Ethyl-3-methylpropyl;
- C₁-C₆-Alkyl: C₂-C₆-Alkyl, wie voranstehend genannt, sowie Methyl;
- C₁-C₄-Halogenalkyl: einen C₁-C₄-Alkylrest, wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, 2-Fluorethyl, 2-Chlorethyl, 2-Bromethyl, 2-Iodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl oder Nonafluorbutyl;
- C₁-C₆-Halogenalkyl: C₁-C₄-Halogenalkyl wie voranstehend genannt, sowie 5-Fluorpentyl, 5-Chlorpentyl, 5-Brompentyl, 5-Iodpentyl, Undecafluorpentyl, 6-Fluorhexyl, 6-Chlorhexyl, 6-Bromhexyl, 6-Iodhexyl oder Dodecafluorhexyl;
- C₁-C₄-Alkoxy: Methoxy, Ethoxy, Propoxy, 1-Methylethoxy, Butoxy, 1-Methylpropoxy, 2-Methylpropoxy oder 1,1-Dimethylethoxy;
- C₁-C₆-Alkoxy: C₁-C₄-Alkoxy wie voranstehend genannt, sowie Pentoxy, 1-Methylbutoxy, 2-Methylbutoxy, 3-Methylbutoxy, 1,1-Dimethylpropoxy, 1,2-Dimethylpropoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Dimethylbutoxy, 1,2-Dimethylbutoxy, 1,3-Dimethylbutoxy, 2,2-Dimethylbutoxy, 2,3-Dimethylbutoxy, 3,3-Dimethylbutoxy, 1-Ethylbutoxy, 2-Ethylbutoxy, 1,1,2-Trimethylpropoxy, 1,2,2-Trimethylpropoxy, 1-Ethyl-1-methylpropoxy oder 1-Ethyl-2-methylpropoxy;
- C₁-C₆-Alkylthio: z.B. Methylthio, Ethylthio, Propylthio, 1-Methylethylthio, Butylthio, 1-Methylpropylthio, 2-Methylpropylthio, 1,1-Dimethylethylthio Pentylthio, 1-Methylbutylthio, 2-Methylbutylthio, 3-Methylbutylthio, 2,2-Dimethylpropylthio, 1-Ethylpropylthio, Hexylthio, 2,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutylthio, 2,3-Dimethylbutylthio, 3,3-Dimethylbutylthio, 1-Ethylbutylthio, 2-Ethylbutylthio, 1,1,2-Trimethylpropylthio, 1,2,2-Trimethylpropylthio, 1-Ethyl-1-methylpropylthio oder 1-Ethyl-2-methylpropylthio;
- C₁-C₆-Alkylsulfonyl (C₁-C₆-Alkyl-S(=O)₂-): z.B. Methylsulfonyl, Ethylsulfonyl, Propylsulfonyl, 1-Methylethylsulfonyl, Butylsulfonyl, 1-Methylpropylsulfonyl, 2-Methylpropylsulfonyl oder 1,1-Dimethylethylsulfonyl, Pentylsulfonyl, 1-Methylbutylsulfonyl, 2-Methylbutylsulfonyl, 3-Methylbutylsulfonyl, 2,2-Dimethylpropylsulfonyl, 1-Ethylpropylsulfonyl, 1,1-Dimethylpropylsulfonyl, 1,2-Dimethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfönyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl oder 1-Ethyl-2-methylpropylsulfonyl; pent-4-en-1-yl, 1,1-Dimethyl-but-2-en-1-yl, 1,1-Dimethyl-but-3-en-1-yl, 1,2-Dimethyl-but-1-en-1-yl, 1,2-Dimethyl-but-2-en-1-yl, 1,2-Dimethyl-but-3-en-1-yl, 1,3-Dimethyl-but-1-en-1-yl, 1,3-Dimethyl-but-2-en-1-yl, 1,3-Dimethyl-but-3-en-1-yl, 2,2-Dimethyl-but-3-en-1-yl, 2,3-Dimethyl-but-1-en-1-yl, 2,3-Dimethyl-but-2-en-1-yl, 2,3-Dimethyl-but-3-en-1-yl, 3,3-Dimethyl-but-1-en-1-yl, 3,3-Dimethyl-but-2-en-1-yl, 1-Ethyl-but-1-en-l-yl, 1-Ethyl-but-2-en-1-yl, 1-Ethyl-but-3-en-1-yl, 2-Ethyl-but-1-en-1-yl, 2-Ethyl-but-2-en-1-yl, 2-Ethyl-but-3-en-1-yl, 1,1,2-Trimethyl-prop-2-en-1-yl, 1-Ethyl-1-methyl-prop-2-en-1-yl, 1-Ethyl-2-methyl-prop-1-en-1-yl oder 1-Ethyl-2-methyl-prop-2-en-1-yl;
- C₂-C₆-Alkenyl: C₃-C₆-Alkenyl wie voranstehend genannt, sowie Ethenyl;
- C₃-C₆-Alkenyloxy: z.B. Prop-1-en-1-yloxy, Prop-2-en-1-yloxy, 1-Methylethenyloxy, Buten-1-yloxy, Buten-2-yloxy, Buten-3-yloxy, 1-Methyl-prop-1-en-1-yloxy, 2-Methyl-prop-1-en-1-yloxy, l-Methyl-prop-2-en-1-yloxy, 2-Methyl-prop-2-en-1-yloxy, Penten-1-yloxy, Penten-2-yloxy, Penten-3-yloxy, Penten-4-yloxy, 1-Methyl-but-1-en-1-yloxy, 2-Methyl-but-1-en-1-yloxy, 3-Methyl-but-1-en-1-yloxy, 1-Methyl-but-2-en-1-yloxy, 2-Methyl-but-2-en-1-yloxy, 3-Methyl-but-2-en-1-yloxy, 1-Methyl-but-3-en-1-yloxy, 2-Methyl-but-3-en-1-yloxy, 3-Methyl-but-3-en-1-yloxy, 1,1-Dimethyl-prop-2-en-1-yloxy, 1,2-Dimethyl-prop-1-en-1-yloxy, 1,2-Dimethyl-prop-2-en-1-yloxy, 1-Ethyl-prop-1-en-2-yloxy, 1-Ethyl-prop-2-en-1-yloxy, Hex-1-en-1-yloxy, Hex-2-en-1-yloxy, Hex-3-en-1-yloxy, Hex-4-en-1-yloxy, Hex-5-en-1-yloxy, 1-Methyl-pent-1-en-1-yloxy, 2-Methyl-pent-1-en-1-yloxy, 3-Methyl-pent-1-en-1-yloxy, 4-Methyl-pent-1-en-1-yloxy, 1-Methyl-pent-2-en-1-yloxy, 2-Methyl-pent-2-en-1-yloxy, 3-Methyl-pent-2-en-1-yloxy, 4-Methyl-pent-2-en-1-yloxy, 1-Methyl-pent-3-en-1-yloxy, 2-Methyl-pent-3-en-1-yloxy, 3-Methyl-pent-3-en-l-yloxy, 4-Methyl-pent-3-en-1-yloxy, 1-Methyl-pent-4-en-1-yloxy, 2-Methyl-pent-4-en-1-yloxy, 3-Methyl-pent-4-en-1-yloxy, 4-Methyl-pent-4-en-1-yloxy, 1,1-Dimethyl-but-2-en-1-yloxy, 1,1-Dimethyl-but-3-en-1-yloxy, 1,2-Dimethyl-but-1-en-1-yloxy, 1,2-Dimethyl-but-2-en-1-yloxy, 1,2-Dimethyl-but-3-en-1-yloxy, 1,3-Dimethyl-but-1-en-1-yloxy, 1,3-Dimethyl-but-2-en-1-yloxy, 1,3-Dimethyl-but-3-en-1-yloxy, 2,2-Dimethyl-but-3-en-1-yloxy, 2,3-Dimethyl-but-1-en-1-yloxy, 2,3-Dimethyl-but-2-en-1-yloxy, 2,3-Dimethyl-but-3-en-1-yloxy, 3,3-Dimethyl-but-1-en-1-yloxy, 3,3-Dimethyl-but-2-en-1-yloxy, 1-Ethyl-but-1-en-1-yloxy, 1-Ethyl-but-2-en-1-yloxy, 1-Ethyl-but-3-en-1-yloxy, 2-Ethyl-but-1-en-1-yloxy, 2-Ethyl-but-2-en-1-yloxy, 2-Ethyl-but-3-en-1-yloxy, 1,1,2-Trimethyl-prop-2-en-1-yloxy, 1-Ethyl-1-methyl-prop-2-en-1-yloxy, 1-Ethyl-2-methyl-prop-1-en-1-yloxy oder l-Ethyl-2-methyl-prop-2-en-1-yloxy;
- C₃-C₆-Alkinyl: Prop-1-in-1-yl, Prop-2-in-1-yl, But-1-in-1-yl, But-1-in-3-yl, But-1-in-4-yl, But-2-in-1-yl, Pent-1-in-l-yl, Pent-1-in-3-yl, Pent-1-in-4-yl, Pent-1-in-5-yl, Pent-2-in-1-yl, Pent-2-in-4-yl, Pent-2-in-5-yl, 3-Methyl-but-1-in-3-yl, 3-Methyl-but-1-in-4-yl, Hex-1-in-1-yl, Hex-1-in-3-yl, Hex-1-in-4-yl, Hex-1-in-5-yl, Hex-1-in-6-yl, Hex-2-in-1-yl, Hex-2-in-4-yl, Hex-2-in-5-yl, Hex-2-in-6-yl, Hex-3-in-1-yl, Hex-3-in-2-yl, 3-Methyl-pent-1-in-1-yl, 3-Methyl-pent-1-in-3-yl, 3-Methyl-pent-1-in-4-yl, 3-Methyl-pent-1-in-5-yl, 4-Methyl-pent-1-in-1-yl, 4-Methyl-pent-2-in-4-yl oder 4-Methyl-pent-2-in-5-yl;
- C₂-C₆-Alkinyl: C₃-C₆-Alkinyl, wie voranstehend genannt, sowie Ethinyl:
- C₃-C₆-Alkinyloxy: z.B. Prop-1-in-1-yloxy, Prop-2-in-1-yloxy, But-1-in-1-yloxy, But-1-in-3-yloxy, But-1-in-4-yloxy, But-2-in-1-yloxy, Pent-1-in-1-yloxy, Pent-1-in-3-yloxy, Pent-1-in-4-yloxy Pent-1-in-5-yloxy, Pent-2-in-1-yloxy, Pent-2-in-4-yloxy, Pent-2-in-5-yloxy, 3-Methyl-but-1-in-3-yloxy, 3-Methyl-but-1-in-4-yloxy, Hex-1-in-1-yloxy, Hex-1-in-3-yloxy, Hex-1-in-4-yloxy, Hex-1-in-5-yloxy, Hex-1-in-6-yloxy, Hex-2-in-1-yloxy, Hex-2-in-4-yloxy, Hex-2-in-5-yloxy, Hex-2-in-6-yloxy, Hex-3-in-1-yloxy, Hex-3-in-2-yloxy, 3-Methylpent-1-in-1-yloxy, 3-Methyl-pent-1-in-3-yloxy, 3-Methyl-pent-1-in-4-yloxy, 3-Methyl-pent-1-in-5-yloxy, 4-Methyl-pent-1-in-1-yloxy, 4-Methyl-pent-2-in-4-yloxy oder 4-Methylpent-2-in-5-yloxy;
- C₃-C₆-Cycloalkyl: Cyclopropyl, Cyclobutyl, Cyclopentyl oder Cyclohexyl;
- C₃-C₆-Cycloalkoxy: Cyclopropoxy, Cyclobutoxy, Cyclopentoxy oder Cyclohexoxy;
- C₃-C₆-Cycloalkenyl: Cyclopropen-1-yl, Cyclobuten-1-yl, Cyclobuten-3-yl, Cyclopenten-1-yl, Cyclopenten-3-yl, Cyclohexen-1-yl, Cyclohexen-3-yl oder Cyclohexen-4-yl;
- Heterocyclyl, sowie die Heteroxyclylreste in Heterocyclyloxy und Heterocyclyl-C₁-C₄-alkyl: drei- bis siebengliedrige, gesättigte oder partiell ungesättigte mono- oder polycyclische Heterocyclen, die ein bis drei Heteroatome ausgewählt aus einer Gruppe bestehend aus Sauerstoff, Stickstoff und Schwefel enthalten, wie Oxiranyl, 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofuran-2-yl, 2,3-Dihydrofuran-3-yl, 2,3-Dihydrofuran-4-yl, 2,3-Dihydrofuran-5-yl, 2,5-Dihydrofuran-2-yl, 2,5-Dihydrofuran-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,5-Dihydroisoxazol-3-yl, 2,5-Dihydroisoxazol-4-yl, 2,5-Dihydroxazol-5-yl, 2,3-Dihydroisothiazol-3-yl, 2,3-Dihydroisothiazol-4-yl, 2,3-Dihydroisothiazol-5-yl, 4,5-Dihydroisothiazol-3-yl, 4,5-Dihydroisothiazol-4-yl, 4,5-Dihydroisothiazol-5-yl, 2,5-Dihydroisothiazol-3-yl, 2,5-Dihydroisothiazol-4-yl, 2,5-Dihydroisothiazol-5-yl, 2,3-Dihydropyrazol-3-yl, 2,3-Dihydropyrazol-4-yl, 2,3-Dihydropyrazol-5-yl, 4,5-Dihydropyrazol-3-yl, 4,5-Dihydropyrazol-4-yl, 4,5-Dihydropyrazol-5-yl, 2,5-Dihydropyrazol-3-yl, 2,5-Dihydropyrazol-4-yl, 2,5-Dihydropyrazol-5-yl, 2,3-Dihydrooxazol-2-yl, 2,3-Dihydrooxazol-4-yl, 2,3-Dihydrooxazol-5-yl, 4,5-Dihydrooxazol-2-yl, 4,5-Dihydrooxazol-4-yl, 4,5-Dihydrooxazol-5-yl, 2,5-Dihydrooxazol-2-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 2-Tetrahydropyranyl, 3-Tetrahydropyranyl, 4-Tetrahydropyranyl, 2-Tetrahydrothiopyranyl, 3-Tetrahydrothiopyranyl, 4-Tetrahydrothiopyranyl, 1,3-Dioxolan-2-yl, 1,3-Dioxolan-4-yl, 1,3-Dithiolan-2-yl, 1,3-Dithiolan-4-yl, 1,3-Dioxan-2-yl, 1,3-Dioxan-4-yl, 1,3-Dioxan-5-yl, 1,3-Dithian-2-yl, 1,3-Dithian-4-yl, 1,3-Dithian-5-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl oder 1,3-Dihydrooxazin-2-yl;
- Hetaryl, sowie die Hetarylreste in Hetaryloxy und Hetaryl-C₁-C₄-alkyl: aromatische mono- oder polycyclische Reste, welche neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl, 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl, 1,2,4,5-Tetrazin-3-yl, sowie die entsprechenden benzokondensierten Derivate;
- C₂-C₆-Alkandiyl: z.B. Ethan-1,2-diyl, Propan-1,3-diyl, Butan-1,4-diyl, Pentan-1,5-diyl oder Hexan-1,6-diyl;

Alle zuvor genannten Phenyl-, Hetaryl- und Heterocyclylringe können substituiert oder unsubstituiert sein. Sie sind vorzugsweise unsubstituiert. Substituierte Ringe tragen ein bis drei Halogenatome und/oder einen oder zwei Reste aus folgender Gruppe: Nitro, Cyano, C₁-C₆-Alkyl, Halogen-C₁-C₆-alkyl, C₁-C₆-Alkoxy, Halogen-C₁-C₆-alkoxy oder C₁-C₆-Alkoxycarbonyl, wie z.B. Methyl, Trifluormethyl, Methoxy, Trifluormethoxy oder Methoxycarbonyl.

Vorläuferverbindungen ("precursors") von Verbindungen I sind beispielsweise Ester- oder Ether-Derivate von funktionellen Hydroxygruppen. In diesem Sinne sollen darunter insbesondere solche Verbindungen der Formel I verstanden werden, in denen die Hydroxygruppe am Pyrazolring ersetzt ist durch die folgenden Reste: C₁-C₆-Alkoxy, C₃-C₆-Alkenyloxy, C₁-C₆-Alkylsulfonyloxy, C₁-C₆-Alkylcarbonyloxy, Phenoxy-, Phenyl-C₁-C₄-alkoxy, Phenylcarbonyl-C₁-C₄-alkoxy, Phenylsulfonyloxy, wobei der Phenylrest der vier letztgenannten Substituenten partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy.

In Hinblick auf die Verwendung der erfindungsgemäßen Verbindungen der Formel I als Herbizide haben die Variablen vorzugsweise folgende Bedeutungen, und zwar jeweils für sich allein oder in Kombination miteinander, wobei im folgenden unter Punkte b) einige Substituenten beispielhaft aufgezählt werden, die unter die unter Punkt a) genannten allgemeinen Begriffe fallen:
1. R¹:
   a) C₁-C₆-Alkyl, C₁-C₆-Alkoxy, Halogen
   b) Methyl, Methoxy, Chlor
2. R² steht vorzugsweise in para-Stellung zur Pyrazolylcarbonylgruppe und bedeutet insbesondere:
   a) C₁-C₆-Alkyl-sulfonyl
   b) Methylsulfonyl, Ethylsulfonyl
3. R⁷
   a) Wasserstoff, C₁-C₆-Alkyl
   b) Wasserstoff, Methyl, Ethyl
4. R⁸
   a) C₁-C₆-Alkyl
   b) Methyl, i-Propyl
5. R⁹
   a) C₁-C₆-Alkyl
   b) Methyl, i-Propyl

Bevorzugte Ausführungsformen sind solche, die mindestens eines oder mehrere der oben genannten Merkmale (s. Punkte 1.-8.) aufweisen.

Besonders bevorzugt sind Verbindungen der Formel I mit folgenden Kombinationen der Reste R⁷, R⁸ und R⁹:
a) R⁷=Wasserstoff; R⁸=R⁹-C₁-C₆-Alkyl
b) R⁷=R⁸=R⁹=C₁-C₆-Alkyl

Eine vorteilhafte Steigerung der herbiziden Wirkung der Benzoylpyrazole kann erfindungsgemäß dadurch erreicht werden, indem die N¹-Position des Pyrazolylringes durch eine verweigtkettige C₁-C₆-Alkyl-gruppe substituiert ist (α,α'-N-Alkyl substituierte Benzoylpyrazole). Diese Wirkungssteigerung tritt grundsätzlich bei allen Benzoylpyrazolen auf, die wesentliche Teile des in der allgemeinen Formel I dargestellten Biophors aufweisen, der für die herbizide Wirkung dieser Verbindungsklasse verantwortlich ist. In diesem Sinne wird als wesentliches Strukturelement die Benzoylpyrazolgruppe verstanden. Bei den übrigen an diesem System vorhandenen Substituenten kann es sich hierbei weitgehend um beliebige Reste handeln. Bevorzugt sind jedoch solche Benzoylpyrazole, die einen oder mehrere der folgenden biophoren Strukturelemente enthalten: eine C₁-C₆-Alkylsulfonylgruppe in 4-Stellung des Phenylringes; eine Halogen-, C₁-C₆-Alkyl- oder C₁-C₆-Alkoxy-gruppe in 2-Stellung des Phenylringes; eine Hydroxy- oder Alkoxygruppe in 5-Stellung des Pyrazolylringes.

Im Sinne der vorliegenden Erfindung sind bevorzugte Ausführungsformen die folgenden Verbindungen der Formeln Ia1 - Ia4:

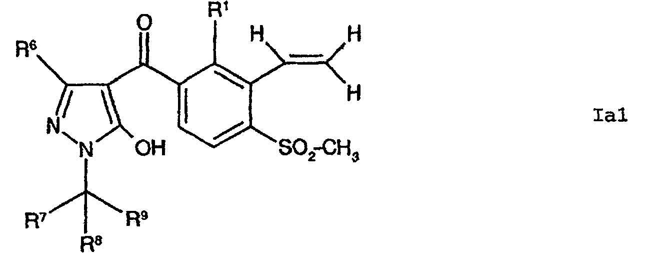

**Tabelle 1**

| Nr. | R¹ | R⁶ | R⁷ | R⁸ | R⁹ |
|---|---|---|---|---|---|
| Ia1.001 | O-CH₃ | H | CH₃ | CH₃ | H |
| Ia1.002 | Cl | H | CH₃ | CH₃ | H |
| Ia1.003 | CH₃ | H | CH₃ | CH₃ | H |
| Ia1.004 | O-CH₃ | H | CH₃ | H | i-C₃H₇ |
| Ia1.005 | Cl | H | CH₃ | H | i-C₃H₇ |
| Ia1.006 | CH₃ | H | CH₃ | H | i-C₃H₇ |
| Ia1.007 | O-CH₃ | H | H | i-C₃H₇ | i-C₃H₇ |
| Ia1.008 | Cl | H | H | i-C₃H₇ | i-C₃H₇ |
| Ia1.009 | CH₃ | H | H | i-C₃H₇ | i-C₃H₇ |
| Ia1.010 | O-CH₃ | H | C₂H₅ | CH₃ | CH₃ |
| Ia1.011 | Cl | H | C₂H₅ | CH₃ | CH₃ |
| Ia1.012 | CH₃ | H | C₂H₅ | CH₃ | CH₃ |

Bevorzugt sind ferner Verbindungen Ia2, insbesondere die Verbindungen Ia2.001-Ia2.012, die sich von den entsprechenden Verbindungen Ia1.001-Ia1.012 dadurch unterscheiden, daß R⁵ in Formel I für Methyl steht:

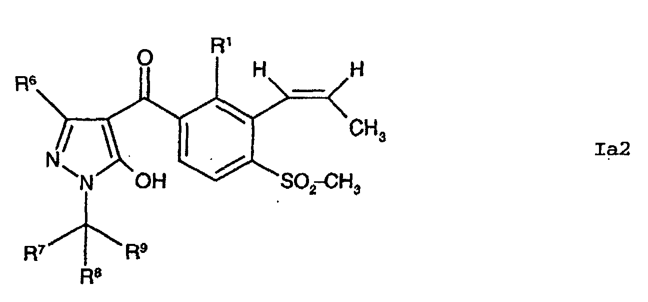

Bevorzugt sind ferner Verbindungen Ia3, insbesondere die Verbindungen Ia3.001-Ia3.012, die sich von den entsprechenden Verbindungen Ia1.001-Ia1.012 dadurch unterscheiden, daß R⁵ in Formel I für Ethyl steht:

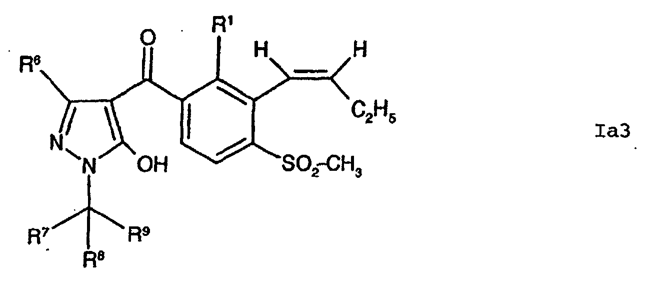

Bevorzugt sind ferner Verbindungen Ia4, insbesondere die Verbindungen Ia4.001-Ia4.012, die sich von den entsprechenden Verbindungen Ia1.001-Ia1.012 dadurch unterscheiden, daß R⁵ in Formel I für i-Propyl steht:

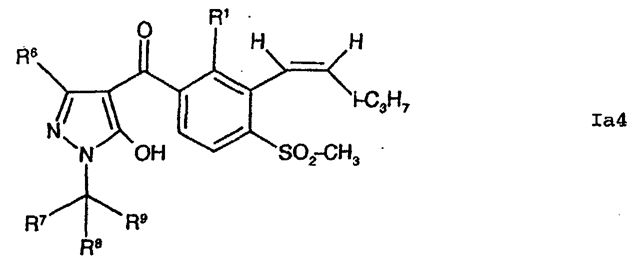

Die Verbindungen der Formel I werden im wesentlichen nach den in WO 98/50366 beschriebenen Verfahren hergestellt.

Besonders eignet sich ein Verfahren, das dadurch gekennzeichnet ist, daß man ein Pyrazol der Formel II 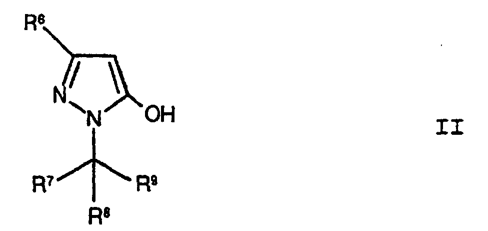 mit einer Carbonsäure III oder einem aktivierten Derivat davon 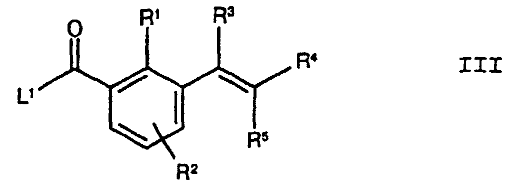 wobei die Variablen R¹ bis R⁵ die unter Anspruch 1 genannten Bedeutungen haben und L¹ für Hydroxy oder eine nucleophil verdrängbare Abgangsgruppe steht, acyliert und das Acylierungsprodukt gegebenenfalls in Gegenwart eines Katalysators zu den Verbindungen I umlagert.

Verbindungen der Formel II sind literaturbekannt oder können käuflich erworben werden. Alternativ können Verbindungen der Formel II nach dem in DE 19910505 beschriebenen Verfahren hergestellt werden.

Verbindungen der Formel III sind unter anderem aus WO 98/50366 und den dort angegebenen Literaturangaben bekannt.

Im folgenden Reaktionsschema wird ein Syntheseweg zur Herstellung von Verbindungen I ausgehend von Verbindungen A über die Zwischenverbindungen B, C und D beispielhaft für R¹ = Methoxy, R²=Methylsulfonyl, R³=R⁴=R⁵=R⁶=H, R⁷=R⁸=CH₃, R⁹=H (Verbindung Ia1.01) beschrieben:

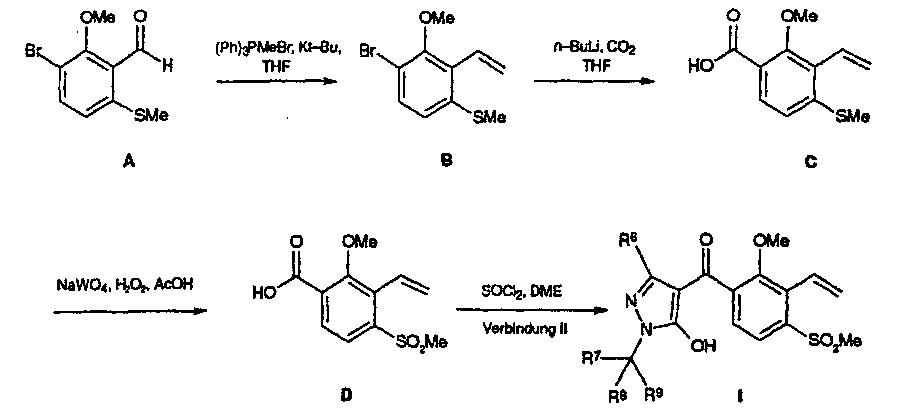

Die Verbindungen I können in Form ihrer landwirtschaftlich brauchbaren Salze vorliegen, wobei es auf die Art des Salzes im allgemeinen nicht ankommt. Üblicherweise werden die Salze von solchen Basen in Betracht kommen, welche die herbizide Wirkung von I nicht negativ beeinträchtigen.

Als basische Salze eignen sich besonders diejenigen der Alkalimetalle, vorzugsweise die Lithium, Natrium- und Kaliumsalze, die der Erdalkalimetalle, vorzugsweise Calcium-, Magnesium-, und Bariumsalze und die der Übergangsmetalle, vorzugsweise Mangan-, Kupfer-, Zink- und Eisensalze, Ammoniumsalze, sowie Ammoniumsalze, die ein bis vier C₁-C₄-Alkyl, Hydroxy-C₁-C₄-alkylsubstituenten, einen Phenyl- oder Benzylsubstituenten tragen können, vorzugsweise Diethylammonium-, Diisopropylammonium-, Tetramethylammonium-, Tetrabutylammonium-, Trimethylbenzylammonium-, und Trimethyl-(2-hydroxyethyl)-ammoniumsalze, die Phosphoniumsalze, die Sulfoniumsalze, vorzugsweise Tri-(C₁-C₄-)alkylsulfoniumsalze, und die Sulfoxoniumsalze, vorzugsweise Tri-(C₁-C₄-)alkylsulfoxoniumsalze.

Die Verbindungen I und deren landwirtschaftlich brauchbaren Salze eignen sich - sowohl als Isomerengemische als auch in Form der reinen Isomeren - als Herbizide. Die die Verbindungen I enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen I bzw. sie enthaltende Mittel noch in einer weiteren Zahl von Kulturpflanzen zur Beseitigung unerwünschter Pflanzen eingesetzt werden. In Betracht kommen beispielsweise folgende Kulturen:

Allium cepa, Ananas comosus, Arachis hypogaea, Asparagus officinalis, Beta vulgaris spec. altissima, Beta vulgaris spec. rapa, Brassica napus var. napus, Brassica napus var. napobrassica, Brassica rapa var. silvestris, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pisum sativum, Prunus avium, Prunus persica, Pyrus communis, Ribes sylvestre, Ricinus communis, Saccharum officinarum, Secale cereale, Solanum tuberosum, Sorghum bicolor (s. vulgare), Theobroma cacao, Trifolium pratense, Triticum aestivum, Triticum durum, Vicia faba, Vitis vinifera, Zea mays.

Darüber hinaus können die Verbindungen I auch in Kulturen, die durch züchtung einschließlich gentechnischer Methoden gegen die Wirkung von Herbiziden tolerant sind, verwandt werden.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Verbindungen I bzw. die sie enthaltenden herbiziden Mittel können beispielsweise in Form von direkt versprübbaren wäßrigen Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Als inerte Zusatzstoffe kommen im Wesentlichen in Betracht: Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Paraffin, Tetrahydronaphthalin, alkylierte Naphthaline oder deren Derivate, alkylierte Benzole oder deren Derivate, Alkohole wie Methanol, Ethanol, Propanol, Butanol, Cyclohexanol, Ketone wie Cyclohexanon oder stark polare Lösungsmittel, z. B. Amine wie N-Methylpyrrolidon oder Wasser.

Wäßrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Verbindungen I als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Lauryletherund Fettalkoholsulfaten, sowie Salze sulfatierter Hexa-, Heptaund Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkaholethylenoxid-Kondensate, ethoxyliertes Rizinusöl, Polyoxyethylenalkylether oder Polyoxypropylenalkylether, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nußschalenmehl, Cellulosepulver oder andere feste Trägerstoffe.

Die Konzentrationen der Wirkstoffe I in den anwendungsfertigen Zubereitungen können in weiten Bereichen variiert werden. Die Formulierungen enthalten im allgemeinen 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Sektrum) eingesetzt.

Die erfindungsgemäßen Verbindungen I können beispielsweise wie folgt formuliert werden:
I 20 Gewichtsteile einer Verbindung I werden in einer Mischung gelöst, die aus 80 Gewichtsteilen alkyliertem Benzol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100.000 Gewichtsteilen Wasser erhält man eine wäßrige Dispersion, die 0,02 Gew. % des Wirkstoffs enthält.
II 20 Gewichtsteile einer Verbindung I werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100.000 Gewichtsteilen Wasser erhält man eine wäßrige Dispersion, die 0,02 Gew.-% des Wirkstoffs enthält.
III 20 Gewichtsteile einer Verbindung I werden in einer Mischung gelöst, die aus 25 Gewichtsteilen Cyclohexanon, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew. % des Wirkstoffs enthält.
IV 20 Gewichtsteile einer Verbindung I werden mit 3 Gewichtsteilen des Natriumsalzes der Diisobutylnaphthalinsulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew. % des Wirkstoffs enthält.
V 3 Gewichtsteile einer Verbindung I werden mit 97 Gewichtsteilen feinteiligem Kaolin vermischt. Man erhält auf diese Weise ein Stäubemittel, das 3 Gew. % des Wirkstoffs enthält.
VI 20 Gewichtsteile einer Verbindung I werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenol-Harnstoff-Formaldehyd-Kondesates und 68 Gewichtsteilen eines paraffinischen Mineralöls innig vermischt. Man erhält eine stabile ölige Dispersion.
VII 1 Gewichtsteil einer Verbindung I wird in einer Mischung gelöst, die aus 70 Gewichtsteilen Cyclohexanon, 20 Gewichtsteilen ethoxyliertem Isooctylphenol und 10 Gewichtsteilen ethoxyliertem Rizinusöl besteht. Man erhält ein stabiles Emulsionskonzentrat.
VIII1 Gewichtsteil einer Verbindung I wird in einer Mischung gelöst, die aus 80 Gewichtsteilen Cyclohexanon und 20 Gewichtsteilen Wettol ® EM 31 (nicht ionischer Emulgator auf der Basis von ethoxyliertem Ricinusöl). Man erhält ein stabiles Emulsionskonzentrat.

Zur Verbreiterung des Wirkungsspektrums und zur Erzielung synergistischer Effekte können die Verbindungen I mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, (Het)-Aryloxyalkansäure und deren Derivate, Benzoesäure und deren Derivate, Benzothiadiazinone, 2-Aroyl-1,3-cyclohexandione, Hetaryl-Aryl-Ketone, Benzylisoxazolidinone, Meta-CF3-phenylderivate, Carbamate, Chinolincarbonsäure und deren Derivate, Chloracetanilide, Cyclohexan-1,3-dionderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Aryloxy- oder Heteroaryloxyphenoxypropionsäureester, Phenylessigsäure und deren Derivate, Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide, Uracile in Betracht.

Außerdem kann es von Nutzen sein, die Verbindungen I allein oder in Kombination mit anderen herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

Die Aufwandmengen an Wirkstoff betragen je nach Bekämpfungsziel, Jahreszeit, Zielpflanzen und Wachstumsstadium 0.001 bis 3.0, vorzugsweise 0.01 bis 1.0 kg/ha aktive Substanz (a. S.)

### Beispiel 1

### (5-Hydroxy-1-isopropyl-1H-pyrazol-4-yl)-(4-methylsulfonyl-2-methoxy-3-vinyl-phenyl)-methanon

a) 2-Methoxy-6-methylsulfanyl-benzaldehyd
   Zu einer Lösung von 2-Chlor-6-methoxybenzaldehyd (20 g, 0.12 mol) in NMP (12.2 g, 0.18 mol) wurde bei 0 °C portionsweise NaSMe (12.2 g, 0.18 mol) gegeben. Die Lösung rührte bei 0 °C 3 h und färbte sich schwarz. Anschließend wurde in ca. 2 l Eiswasser eingerührt, mit 10%iger HCl auf pH = 3 angesäuert und der Niederschlag abgesaugt.
   Ausbeute: 13.6 g (65%). ¹H-NMR(270MHz, CDCl₃) : 2.4 (s, 3H); 3.9 (s, 3H); 6.8 (d, 1H); 6.95 (d, 1H); 7.42 (d, 1H); 10.6 (s, 1H).
b) 3-Brom-2-methoxy-6-methylsulfanyl-benzaldehyd
   Zu einer Lösung von 2-Methoxy-6-methylsulfanyl-benzaldehyd (22 g, 0.12 mol) in Dioxan (500 ml) wurde Brom (28.8 g, 0.18 mol) (in Dioxan (500 ml) gelöst) getropft und bei 50 ° C über 6 h gerührt. Die wurde dann eingeengt, in CH₂Cl₂ aufgenommen, mit H₂O gewaschen, über MgSO₄ getrocknet und eingeengt. Der Feststoff wurde aus Diisopropylether umkristallisiert.
   Ausbeute: 17.5 g (56%). ¹H-NMR(270MHz, CDCl₃): 2.40 (s, 3H); 3.90 (s, 3H); 6.72 (d, 1H); 6.85 (d, 1H); 7.42 (m, 1H); 10.6 (s, 1H).
c) 1-Brom-2-methoxy-4-methylsulfanyl-3-vinyl-benzol
   Zu einer Lösung von Methytriphenylphosphoniumbromid (19.7 g, 55.4 mmol) in THF (180 ml) wurde bei 0 °C K-t-Bu (6.2 g, 55.4 mmol) gegeben. Anschließend wurde bei -10 bis -5 °C 3-Brom-2-methoxy-6-methylsulfanyl-benzaldehyd (12 g, 46 mmol) gelöst in THF (180 ml) zugegeben und 7 h bei RT über Nacht gerührt. Die Mischung wurde filtriert, die Lösung mit H₂O (200 ml) und MTBE (200 ml) versetzt und anschließend mit MTBE (200 ml) extrahiert. Die vereinigten org. Phasen wurden über MgSO₄ getrocknet und eingeengt. Chromatographie (Cyclohexan -> Cylohexan/EtOAc 9:1) lieferte 1-Brom-2-methoxy-4-methylsulfonyl-3-vinyl-benzol.
   Ausbeute: 4.67 g (39%). ¹H-NMR(270MHz, CDCl₃): 2.40 (s, 3H); 3.70 (s, 3H); 5.62 (dd, 1H); 5.90 (dd, 1H); 6.70-6.90 (m, 2H); 7.40 (s, 1H).
d) 2-Methoxy-4-methylsulfanyl-3-vinyl-benzoesäure
   Zu einer Lösung von 1-Brom-2-methoxy-4-methylsulfanyl-3-vinyl-benzol (7 g, 31.3 mmol) in THF (300 ml) wurde bei -100 °C n-BuLi (25 ml, 15%ig in n-Hexan, 4.1 mmol) gegeben und 20 min. bei -100 °C nachgerührt. Anschließend wurde bei -100 °C CO₂ eingeleitet (exoterme Reaktion auf -60 °C). Dann wurde noch 1 h bei -80 bis -90 °C nachgerührt und bei -40 °C NaOH (100 ml) zugetropft. Die Lösung wurde in EtOAc (400 ml) eingerührt. 3 x mit 5%iger NaOH extrahiert, mit 10%iger HCl angesäuert auf pH = 1. Die H₂O-Phase wurde mit EtOAc (300 ml) extrahiert, die vereinigten org. Phasen über MgSO₄ getrocknet und eingeengt.
   Ausbeute: 6.8 g (98%). ¹H-NMR(270MHz, CDCl₃): 1.90 (s, 3H); 2.42 (s, 3H); 3.62 (s, 3H); 5.50-5.80 (m, 2H); 6.58-6.65 (m, 1H); 7.1 (d, 1H); 7.6 (d, 1H).
e) 4-Methylsulfonyl-2-methoxy-3-vinyl-benzoesäure
   Zu einer Lösung von 2-Methoxy-4-methylsulfanyl-3-vinyl-benzoesäure (6.8 g, 30 mmol) in AcOH (130 ml) wurde NaWO₄ (kat.) gegeben, H₂O₂ (8.6 ml, 76 mmol) zugetropft und 5 h bei Raumtemperatur gerührt. Die Lösung wurde eingeengt, in Methylenchlorid aufgenommen, über Magnesiumsulfat getrocknet und eingeengt.
   Ausbeute: 4.8 g (61%). ¹H-NMR(270MHz, CDCl₃): 3.20 (s, 3H); 3.70 (s, 3H); 5.65-5.90 (m, 2H); 7.0-7.20 (m, 1H); 7.65-7.95 (m, 2H).
f) 5-Hydroxy-1-isopropyl-1H-pyrazol-4-yl)-(4-methylsulfonyl-2-methoxy-3-vinyl-phenyl)-methanon
   Zu einer Lösung von 4-Methylsulfonyl-2-methoxy-3-vinyl-benzoesäure (1.6 g, 6 mmol) in Toluol (60 ml) wurde SOCl₂ (1.2 g, 10 mmol) gegeben und 2 h auf Rückfluß erhitzt. Anschließend wurde eingeengt und das Produkt zu einer Lösung von N-Isoproyplpyrazolon (6 mmol), K₂CO₃ (1.6 g, 11.6 mmol) in DME (30 ml) gegeben und bei RT über Nacht gerührt. Anschließend wurde 2 h auf Rückfluß erhitzt, die Mischung eingeengt und in H₂O gelöst. Die H₂O-Phase wurde 3 x mit CH₂Cl₂ extrahiert, die vereinigten org. Phasen wurden über MgSO₄ getrocknet und eingeengt.

### Beispiel 2

In gleicher Weise wie in Beispiel 1 beschrieben können die folgenden Verbindungen erhalten werden:

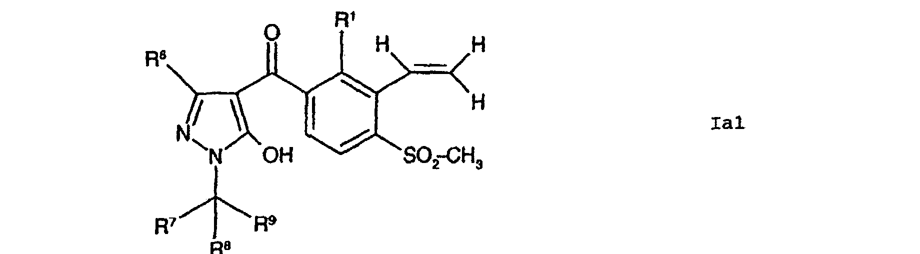

| Bsp. Nr. | R¹ | R⁶ | R⁷ | R⁸ | R⁹ | Fp. |
|---|---|---|---|---|---|---|
| 2.01 | O-CH₃ | H | CH₃ | CH₃ | H | 57-60°C |
| 2.02 | Cl | H | CH₃ | CH₃ | H | |
| 2.03 | CH₃ | H | CH₃ | CH₃ | H | 134-135°C |
| 2.04 | O-CH₃ | H | CH₃ | H | i-C₃H₇ | |
| 2.05 | Cl | H | CH₃ | H | i-C₃H₇ | |
| 2.06 | CH₃ | H | CH₃ | H | i-C₃H₇ | |
| 2.07 | O-CH₃ | H | H | i-C₃H₇ | i-C₃H₇ | |
| 2.08 | Cl | H | H | i-C₃H₇ | i-C₃H₇ | |
| 2.09 | CH₃ | H | H | i-C₃H₇ | i-C₃H₇ | |
| 2.10 | O-CH₃ | H | C₂H₅ | CH₃ | CH₃ | |
| 2.11 | Cl | H | C₂H₅ | CH₃ | CH₃ | |
| 2.12 | CH₃ | H | C₂H₅ | CH₃ | CH₃ | |
| 2.13 | OCH₃ | H | CH₃ | CH₃ | CH₃ | 57-60°C |
| 2.14 | CH₃ | H | CH₃ | CH₃ | CH₃ | 122-124°C |
| 2.15 | OC₂H₅ | H | CH₃ | CH₃ | H | 123-124°C |
| 2.16 | OC₂H₅ | H | CH₃ | CH₃ | CH₃ | 106-108°C |
| 2.17 | Cl | H | CH₃ | CH₃ | CH₃ | 117-125°C |

### Beispiel 3

In gleicher Weise wie in Beispiel 1 beschrieben, können die folgenden Verbindungen erhalten werden:

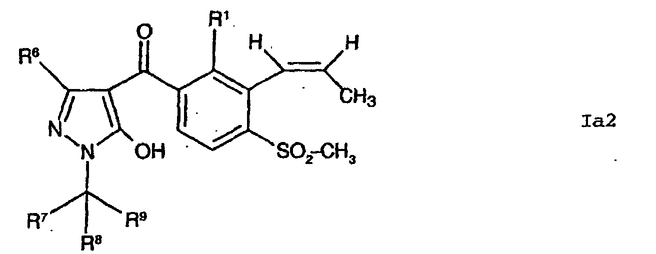

| Bsp. Nr. | R¹ | R⁶ | R⁷ | R⁸ | R⁹ | Fp. |
|---|---|---|---|---|---|---|
| 3.1 | Cl | H | H | CH₃ | CH₃ | 44-54°C |
| 3.2 | CH₃ | H | H | CH₃ | CH₃ | 58-68°C |
| 3.3 | CH₃ | H | CH₃ | CH₃ | CH₃ | 63-74°C |

Die herbizide Wirkung der Verbindungen der Formel I ließ sich durch Gewächshausversuche zeigen:

Als Kulturgefäße dienen Plastiktöpfe mit lehmigem Sand mit etwa 3,0% Humus als Substrat. Die Samen der Testpflanzen werden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandsmenge für die Nachauflaufbehandlung betrug 0,25, 0,125 bzw. 0,0625 kg/ha aktive Substanz (a.S.).

Die Pflanzen wurden artenspezifisch bei Temperaturen von 10 - 25°C bzw. 20 - 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Code | Deutscher Name | Englischer Name |
|---|---|---|
| AMARE | Fuchsschwanz, zurückgekrümmter | pigweed |
| CHEAL | Weißer Gänsefuß | lambsquarter |
| ECHCG | Hühnerhirse | barnyardgrass |
| POLPE | Flohknöterich | ladysthumb |
| SETVI | Grüne Borstenhirse | green foxtail |
| SINAL | Weißer Senf | white mustard |
| SOLNI | Schwarzer Nachtschatten | black nightshade |

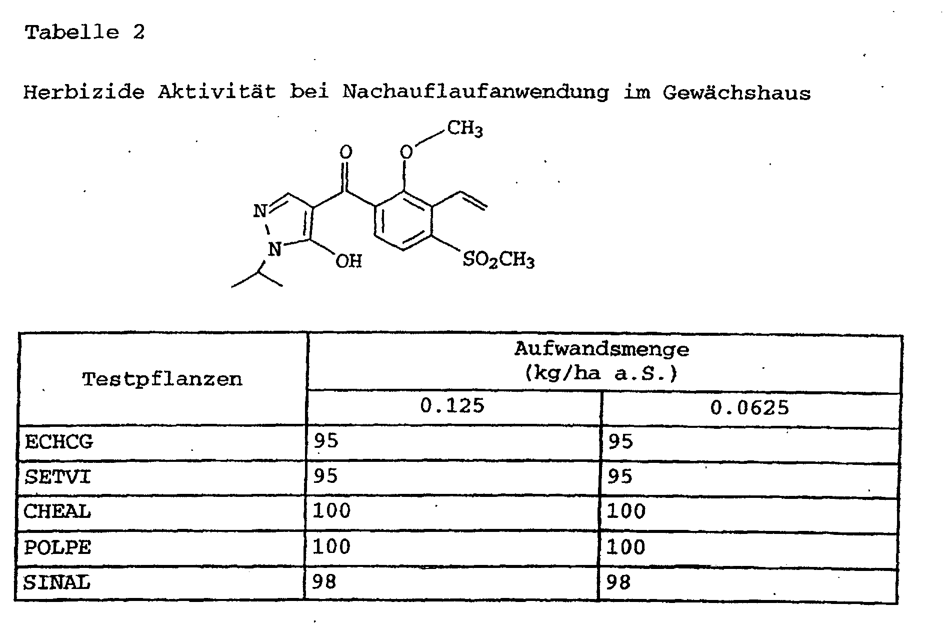

## Patentansprüche

1. Pyrazole der Formel I 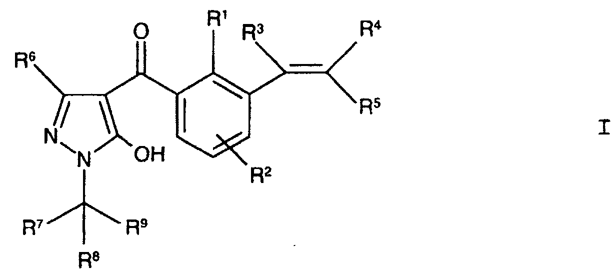 in der die Variablen folgende Bedeutung haben:
R¹ Halogen, C₁-C₆-Alkyl, C₁-C₆-Alkoxy, C₁-C₆-Halogen-alkyl, C₁-C₆-Alkylthio;
R² -S(O)₂R¹⁰, -SO₂OR¹¹;
R³ Wasserstoff;
R⁴, R⁵ Wasserstoff, C₁₋C₆-Alkyl,
R⁶ Wasserstoff;
R⁷, R⁸, R⁹ Wasserstoff, C₁-C₆-Alkyl, wobei die Alkylgruppe gegebenenfalls ein- oder mehrfach durch Halogen oder Cyano substituiert sein kann, und die Reste R⁷, R⁸ und R⁹ jeweils gleich oder verschieden sind, wobei maximal einer der Reste aus der Gruppe R⁷, R⁸, R⁹ Wasserstoff bedeutet;
R¹⁰, R¹¹ C₁-C₆-Alkyl;
sowie deren Tautomere und landwirtschaftlich brauchbaren Salze.

2. Pyrazole nach Anspruch 1, wobei R⁷ Wasserstoff oder C₁-C₆-Alkyl bedeutet.

3. Pyrazole nach Anspruch 1 oder 2, wobei R⁸ C₁-C₆-Alkyl bedeutet.

4. Pyrazole nach einem der Ansprüche 1 - 3, wobei R⁹ C₁-C₆-Alkyl bedeutet.

5. Pyrazole nach einem der Ansprüche 1 - 4, wobei R⁷ Wasserstoff ist und R⁸ und R⁹ C₁-C₆-Alkyl bedeuten.

6. Pyrazole nach einem der Ansprüche 1 - 5, wobei R¹ C₁-C₆-Alkoxy, C₁-C₆-Alkyl oder Halogen bedeutet.

7. Pyrazole nach einem der Ansprüche 1 - 6, wobei R² in para-Stellung zur Pyrazolylcarbonylgruppe steht.

8. Pyrazole nach einem der Ansprüche 1 - 7, wobei R³, R⁴ und R⁵ jeweils gleich sind und Wasserstoff bedeuten.

9. Verfahren zur Herstellung von Pyrazolen gemäß den Ansprüchen 1 - 8, **dadurch gekennzeichnet, daß** man ein Pyrazol der Formel II 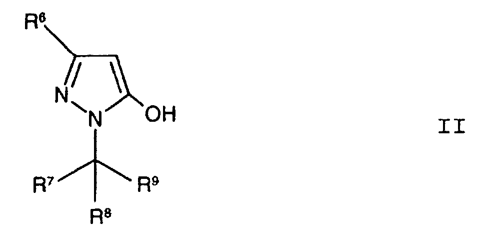 mit einer Carbonsäure III oder einem aktivierten Derivat davon 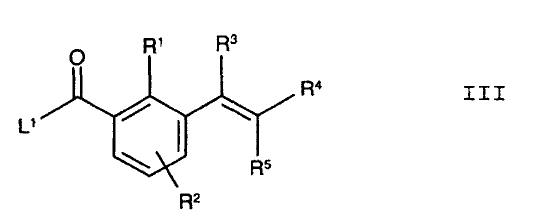 wobei die Variablen R¹ bis R⁵ die unter Anspruch 1 genannten Bedeutungen haben und L¹ für Hydroxy oder eine nucleophil verdrängbare Abgangsgruppe steht, acyliert und das Acylierungsprodukt gegebenenfalls in Gegenwart eines Katalysators zu den Verbindungen I umlagert.

10. Mittel, enthaltend eine herbizid wirksame Menge mindestens eines Pyrazols der Formel I oder eines landwirtschaftlich brauchbaren Salzes von I gemäß den Ansprüchen 1 bis 8 und für die Formulierung von Pflanzenschutzmitteln übliche Hilfsmittel.

11. Verfahren zur Herstellung von herbizid wirksamen Mitteln gemäß Anspruch 14, **dadurch gekennzeichnet, daß** man eine herbizid wirksame Menge mindestens eines Pyrazols der Formel I oder eines landwirtschaftlich brauchbaren Salzes von I gemäß den Ansprüchen 1 bis 8 und für die Formulierung von Pflanzenschutzmitteln übliche Hilfsmittel mischt.

12. Verfahren zur Bekämpfung von unerwünschtem Pflanzenwuchs, **dadurch gekennzeichnet, daß** man eine herbizid wirksame Menge mindestens eines Pyrazols der Formel I oder eines landwirtschaftlich brauchbaren Salzes von I gemäß den Ansprüchen 1 bis 8 auf Pflanzen, deren Lebensraum und/oder auf Samen einwirken läßt.

13. Verwendung der Pyrazole der Formel I und deren landwirtschaftlich brauchbaren Salze gemäß den Ansprüchen 1 bis 8 als Herbizide.

## Claims

1. A pyrazole of the formula I 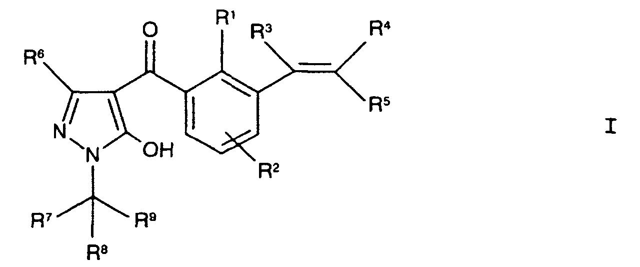 where:
R¹ is halogen, C₁-C₆-alkyl, C₁-C₆-alkoxy, C₁-C₆-haloalkyl, C₁-C₆-alkylthio;
R² is -S(O)₂R¹⁰, -SO₂OR¹¹;
R³ is hydrogen;
R⁴, R⁵ are hydrogen, C₁-C₆-alkyl;
R⁶ is hydrogen;
R⁷, R⁸, R⁹ are hydrogen, C₁-C₆-alkyl, where the alkyl group may be unsubstituted or mono- or polysubstituted by halogen or cyano and the radicals R⁷, R⁸ and R⁹ are in each case identical or different, but at most one of the radicals of the group R⁷, R⁸ and R⁹ is hydrogen;
R¹⁰,R¹¹ are C₁-C₆-alkyl;
and its tautomers and agriculturally useful salts.

2. A pyrazole as claimed in claim 1 where R⁷ is hydrogen or C₁-C₆-alkyl.

3. A pyrazole as claimed in claim 1 or 2 where R⁸ is C₁-C₆-alkyl.

4. A pyrazole as claimed in any of claims 1 - 3 where R⁹ is C₁-C₆-alkyl.

5. A pyrazole as claimed in any of claims 1 - 4 where R⁷ is hydrogen and R⁸ and R⁹ are C₁-C₆-alkyl.

6. A pyrazole as claimed in any of claims 1 - 5 where R¹ is C₁-C₆-alkoxy, C₁-C₆-alkyl or halogen.

7. A pyrazole as claimed in any of claims 1 - 6 where R² is in the para position to the pyrazolylcarbonyl group.

8. A pyrazole as claimed in any of claims 1 - 7 where R³, R⁴ and R⁵ are each identical and are hydrogen.

9. A process for preparing a pyrazole as claimed in any of claims 1 - 8 which comprises acylating a pyrazole of the formula II 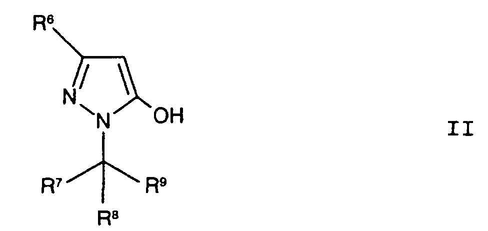 with a carboxylic acid III or an activated derivative thereof 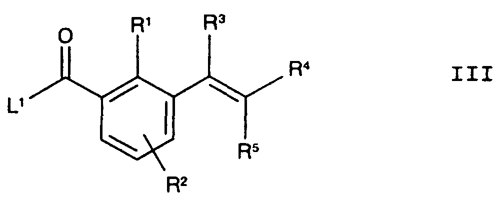 where the variables R¹ to R⁵ are as defined under claim 1 and L¹ is hydroxyl or a nucleophilically displaceable leaving group, and rearranging the acylation product in the presence or absence of a catalyst to the compounds I.

10. A composition comprising a herbicidally effective amount of at least one pyrazole of the formula I or an agriculturally useful salt of I as claimed in any of claims 1 to 8 and auxiliaries which are customary for formulating crop protection agents.

11. A process for preparing a herbicidally active composition as claimed in claim 10 which comprises mixing a herbicidally effective amount of at least one pyrazole of the formula I or an agriculturally useful salt of I as claimed in any of claims 1 to 8 and auxiliaries which are customary for formulating crop protection agents.

12. A method for controlling undesirable vegetation which comprises allowing a herbicidally effective amount of at least one pyrazole of the formula I or an agriculturally useful salt of I as claimed in any of claims 1 to 8 to act on plants, their habitat and/or on seeds.

13. The use of the pyrazoles of the formula I and their agriculturally useful salts as claimed in any of claims 1 to 8 as herbicides.

## Revendications

1. Pyrazoles de formule I 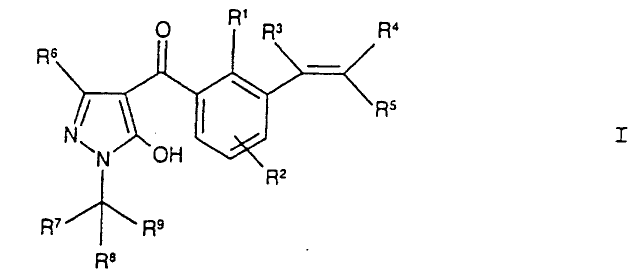 dans laquelle les symboles ont les significations suivantes :
R¹ : un halogène, un groupe alkyle en C1-C6, alcoxy en C1-C6, halogénoalkyle en C1-C6, alkylthio en C1-C6 ;
R² : un groupe -S(O)₂R¹⁰, -SO₂OR¹¹ ;
R³ : l'hydrogène ;
R⁴, R⁵ : l'hydrogène, des groupes alkyle en C1-C6 ;
R⁶ : l'hydrogène ;
R⁷, R⁸, R⁹ : l'hydrogène, des groupes alkyle en C1-C6 qui peuvent le cas échéant être substitués par un ou plusieurs halogènes ou groupes cyano, R⁷, R⁸ et R⁹ pouvant avoir des significations identiques ou différentes, un seul au maximum des symboles R⁷, R⁸, R⁹ représentant l'hydrogène ;
R¹⁰, R¹¹ : des groupes alkyle en C1-C6,
et leurs tautomères et sels aptes aux applications agricoles.

2. Pyrazoles selon la revendication 1 pour lesquels R⁷ représente l'hydrogène ou un groupe alkyle en C1-C6.

3. Pyrazoles selon la revendication 1 ou 2 pour lesquels R⁸ représente un groupe alkyle en C1-C6.

4. Pyrazoles selon l'une des revendications 1 à 3 pour lesquels R⁹ représente un groupe alkyle en C1-C6.

5. Pyrazoles selon l'une des revendications 1 à 4 pour lesquels R⁷ représente l'hydrogène et R⁸ et R⁹ des groupes alkyle en C1-C6.

6. Pyrazoles selon l'une des revendications 1 à 9 pour lesquels R⁹ représente un groupe alcoxy en C1-C6, alkyle en C1-C6 ou un halogène.

7. Pyrazoles selon l'une des revendications 1 à 6 pour lesquels R² est en position para du groupe pyrazolylcarbonyle.

8. Pyrazoles selon l'une des revendications 1 à 7 pour lesquels R³, R⁴ et R⁵ représentent tous l'hydrogène.

9. Procédé pour la préparation des pyrazoles selon les revendications 1 à 8, **caractérisé par le fait que** l'on acyle un pyrazole de formule II 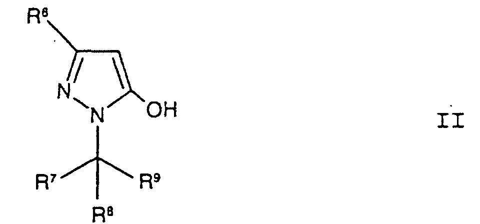 à l'aide d'un acide carboxylique III ou d'un dérivé activé de cet acide 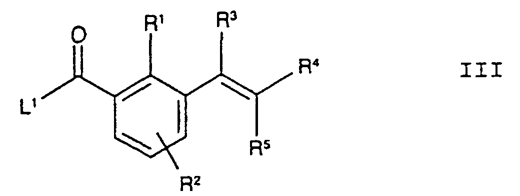 les symboles R¹ à R⁵ ayant les significations indiquées dans la revendication 1 et L¹ représentant un groupe hydroxy ou un groupe éliminable par échange nucléophile, et on soumet le produit d'acylation à transposition en les composés I, éventuellement en présence d'un catalyseur.

10. Produit contenant une quantité herbicide efficace d'au moins un pyrazole de formule I ou de l'un de ses sels acceptables pour les usages agricoles selon les revendications 1 à 8 et des produits auxiliaires usuels pour la formation des produits phytosanitaires.

11. Procédé pour la préparation de produits à activité herbicide selon la revendication 14, **caractérisé par le fait que** l'on mélange une quantité herbicide efficace d'au moins un composé de formule I ou de l'un de ses sels aptes aux applications agricoles selon les revendications 1 à 8 et des produits auxiliaires usuels pour la formation des produits phytosanitaires.

12. Procédé pour combattre les croissances de végétaux indésirables, **caractérisé par le fait que** l'on fait agir une quantité efficace d'au moins un pyrazole de formule I ou de l'un de ses sels aptes aux applications agricoles selon les revendications 1 à 8 sur les végétaux, leur habitat et/ou sur les semences.

13. Utilisation des pyrazoles de formule I et de leurs sels aptes aux applications agricoles selon les revendications 1 à 8 en tant qu'herbicides.
